# EUROPÄISCHE PATENTSCHRIFT

(11) **EP 1 028 977 B1**
(45) Veröffentlichungstag und Bekanntmachung des Hinweises auf die Patenterteilung: **26.11.2003**
(21) Anmeldenummer: 98951420.3
(22) Anmeldetag: 16.09.1998
(51) Int. Cl.: C07K 14/79, C07K 14/47, C07K 14/765, C07K 14/705, C12N 9/36, A23L 1/305, C07K 5/103, C07K 7/06, C07K 16/04, C12N 15/12, G01N 33/68, A61K 38/07, A61K 38/08, A61K 38/17, A61K 38/40, A61K 38/38, A61K 38/47, A61P 31/00

(54) **BIFIDOGENE PEPTIDE UND DEREN VERWENDUNG**
BIFIDUS STIMULATING PEPTIDES AND THEIR USES
PEPTIDES BIFIDOGENES ET LEURS UTILISATIONS

(30) Priorität: 16.09.1997 DE 19740604; 11.02.1998 DE 19805385
(43) Veröffentlichungstag der Anmeldung: 23.08.2000
(73) Patentinhaber: FORSSMANN, Wolf-Georg, D-30625 Hannover (DE)
(72) Erfinder: FORSSMANN, Wolf-Georg, D-30625 Hannover (DE); ZUCHT, Hans-Dieter, D-30625 Hannover (DE); LIEPKE, Cornelia, D-30625 Hannover (DE)
(74) Vertreter: Meyers, Hans-Wilhelm, Dr.Dipl.-Chem.
(86) Internationale Anmeldenummer: EP9805899
(87) Internationale Veröffentlichungsnummer: WO99014231

(56) Entgegenhaltungen:
- WO-A-90/13642
- BELLAMY W ET AL: "IDENTIFICATION OF THE BACTERICIDAL DOMAIN OF LACTOFERRIN" BIOCHIMICA ET BIOPHYSICA ACTA,NL,AMSTERDAM, Bd. 1121, 1992, Seiten 130-136, XP000886628 ISSN: 0006-3002
- BELLAMY E A: "Antibacterial spectrum of lactoferricin B, a potent bactericidal peptide derived from the N-terminal region of bovine lactoferrin" JOURNAL OF BACTERIOLOGY,US,WASHINGTON, DC, Bd. 73, Nr. 6, 1992, Seiten 472-479, XP002094251 ISSN: 0021-9193
- ODELL E.W. ET AL: 'Antibacterial activity of peptides homologous to a loop region in human lactoferrin' FEBS LETTERS Bd. 382, Nr. 1-2, 1996, Seiten 175 - 178

### SEQUENZPROTOKOLL

(1) ALLGEMEINE ANGABEN:
   (i) ANMELDER:
      (A) NAME: Wolf-Georg Forssmann
      (B) STRASSE: Feodor-Lynen-Strasse 31
      (C) ORT: Hannover
      (E) LAND: Deutschland
      (F) POSTLEITZAHL: 30625
   (ii) BEZEICHNUNG DER ERFINDUNG: Bifidogene Peptide
   (iii) ANZAHL DER SEQUENZEN: 24
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 8:
   (i) SEQUENZKENNZEICHEN:
      (A) LÄNGE: 10 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (xi) SEQUENZBESCHREIBUNG: SEQ ID NO: 8:
   Glu Val Ala Ala Arg Ala Arg Val Val Trp
   1               5                   10
(2) ANGABEN ZU SEQ ID NO: 14:
   (i) SEQUENZKENNZEICHEN:
      (A) LÄNGE: 12 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (xi) SEQUENZBESCHREIBUNG: SEQ ID NO: 14:
   Ala Arg Arg Ala Arg Val Val Trp Cys Ala Val Gly
   1               5                   10
(2) ANGABEN ZU SEQ ID NO: 16:
   (i) SEQUENZKENNZEICHEN:
      (A) LÄNGE: 13 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (xi) SEQUENZBESCHREIBUNG: SEQ ID NO: 16:
   Ala Arg Arg Ala Arg Val Val Trp Cys Ala Val Gly Glu
   1               5                   10
(2) ANGABEN ZU SEQ ID NO: 22:
   (i) SEQUENZKENNZEICHEN:
      (A) LÄNGE: 12 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (xi) SEQUENZBESCHREIBUNG: SEQ ID NO: 22:
   Ala Arg Arg Ala Arg Val Val Trp Ala Ala Val Gly
   1               5                   10

## Beschreibung

Die vorliegende Erfindung betrifft bifidogene Peptide, Arzneimittel enthaltend die erfindungsgemäßen Peptide sowie die Verwendung der bifidogenen Peptide.

Von Milch ist bekannt, dass sie fördernd auf den Gesundheitszustand von Säuglingen wirkt. Dies wird vielfach auf den Einfluss der Milch auf die Ausbildung einer säuglingstypischen Darmflora zurückgeführt, die zu mehr als 90% aus Bifidobacterium bifidum besteht.

WO 90/13642 A offenbart die Sequenz des humanen Lactoferrins.

In Bellamy e.a.: "Antibacterial spectrum of lactoferricin B, a potent bactericidal peptide derived from the N-terminal region of bovine lactoferrin", Journal of Applied Bacteriology, Bd. 73, Nr. 6, 1992, Seiten 472 bis 479 und Odell e.w. et al.: " Antibacterial activity of peptides homologous to a loop region in human lactoferrin", FEBS Letters, Bd. 382, Nr. 1 bis 2, 1996, Seiten 175 bis 178 wird die antibacterielle Aktivität der Peptide Lactoferricin B und H, die aus bovinen oder humanen Lactoferrinen stammen, offenbart.

Aufgabe der vorliegenden Erfindung ist es Peptide bereitzustellen, die einen positiven Einfluss auf die Darmflora haben.

Die erfindungsgemäße Aufgabe wird gelöst durch Peptide mit den Merkmalen des Patentanspruchs 1.

Die erfindungsgemäßen Peptide wirken antimikrobiell gegen Bakterien, die in der natürlichen Säuglingsdarmflora nicht oder nur in geringen Mengen vorkommen und fördern das Wachstums von erwünschten Bakterien wie Bifidobakterien, indem sie die Bifidobakterien im Wachstum stärker als andere Bakterien fördern oder nur die nichterwünschten Bakterien selektiv hemmen. Diese Eigenschaft, Bifidobakterien einen Wachstumsvorteil zu verschaffen, wird als bifidogen bezeichnet.

Die erfindungsgemäßen Peptide weisen die folgende Aminosäuresequenz auf:

R¹-EVAARARVVW-R²,

R¹-ARRARVVWAAVG-R²,

R¹-ARRARVVWCAVGE-R²,

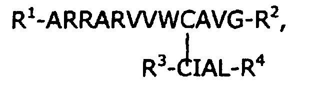

worin
R¹, R³ unabhängig voneinander NH₂, eine Aminosäure oder ein Peptid mit bis 100 Aminosäuren darstellen und
R², R⁴ unabhängig voneinander COOH, CONH₂, eine Aminosäure oder ein Peptid mit bis zu 100 Aminosäuren darstellen
sowie die amidierten, acetylierten, sulfatierten, phosphorylierten, glycosylierten, oxydierten Derivate oder Fragmente mit bifidogenen Eigenschaften und Peptide, die durch Kombination der Peptide, Fragmente oder Derivate mittels chemischer Verknüpfung erhältlich sind.

Bevorzugt weisen R₁, R₂, R₃, R₄ eine Länge von bis zu 50, mehr bevorzugt von bis zu 20 und am meisten bevorzugt eine Länge von bis zu 10 Aminosäuren auf.

Die erfindungsgemäßen Peptide können durch Aufreinigung aus Kuhmilch oder Humanmilch erhalten werden. Alternativ ist jedoch auch die Expression in gentechnisch veränderten Organismen oder die Herstellung durch chemische Peptidsynthese möglich.

Ein weiterer Aspekt der Erfindung sind Nucleinsäuren, die für die bifidogenen Peptide kodieren.

Die erfindungsgemäßen Peptide und/oder Nucleinsäuren können zusammen mit pharmazeutisch üblichen Hilfsstoffen in Arzneimitteln enthalten sein. Dazu werden bevorzugt galenische Zubereitungen eingesetzt und Applikationsformen gewählt, bei denen die Peptide undegradiert an den Wirkort gelangen.

Vorzugsweise wurden die erfindungsgemäßen Peptide in Mengen von 0,1 bis 100 mg/kg Körpergewicht eingesetzt. Wirksame Nucleinsäuremengen sind beispielsweise 0,01 mg bis 100 mg/kg Körpergewicht. Bevorzugt liegt die Menge im Bereich von 1 bis 10 mg/kg Körpergewicht für die Peptide und Nucleinsäuren.

Die erfindungsgemäßen Peptide können auch zusammen mit Nährstoffen in Nahrungsmittel enthalten sein.

Darüber hinaus können die erfindungsgemäßen Peptide und/oder die gegen die Peptide gerichteten Antikörper zusammen mit weiteren Hilfsstoffen auch in Diagnostikmittel enthalten sein.

Die erfindungsgemäßen Peptide und Nukleinsäuren eignen sich zur Behandlung von durch mikrobielle Fehlbesiedlungen bedingte Erkrankungen wie Infektionen, Entzündungen, mikrobiell induzierten Tumoren, mikrobiell bedingten degenerativen Erkrankungen, Durchfallerkrankungen, Koliken, Abweichung der Mund-, Darm- und Vaginalflora, Karies. Die mikrobielle Fehlbesiedlung kann beispielsweise durch Bakterien, Pilze, Hefen, Protisten, Viren, Mycoplasmen, Filarien und/oder Plasmodien bedingt sein.

Die erfindungsgemäßen Peptide eignen sich auch als Hilfsstoff in der Nahrungsmittelzubereitung im Sinne von Fermentationshilfen.

Es wird insbesondere bevorzugt, dass zwei oder mehr Peptide gemeinsam eingesetzt werden oder Peptide eingesetzt werden, die zwei oder mehr der erfindungsgemäßen Peptidsequenzen aufweisen. Das unterschiedliche Wirkungsspektrum der Einzelstoffe bzw. Stoffe, die Einzelsequenzen oder erfindungsgemäße Peptide aufweisen, erlaubt es, beim Vorliegen von Resistenzen von Mikroorganismen durch eine geeignete Kombination von Sequenzen oder durch eine Kombination der Einzelsubstanzen eine optimale Hemmung der unerwünschten Mikroorganismen zu erzielen.

### Die folgenden Beispiele sollen die Erfindung weiter erläutern:

### Referenzbeispiel 1

### Behandlung von Milch

Humane Milch wurde mit Pepsin (20 mg/g Protein) versetzt, nachdem sie mit HCl auf einen pH von 3,5 eingestellt wurde. Die enzymatische Reaktion wurde für zwei Stunden bei 37°C inkubiert und durch Kochen für fünf Minuten gestoppt. Anschließend wurde zentrifugiert (20 min, 60.000 g bei 4°C) und das Milchfett abgeschöpft. Die verbliebene Lösung wurde mit 0,1% TFA versetzt und erneut zentrifugiert, um ausgefallene hochmolekulare Proteine abzutrennen.

### HPLC Reinigung eines bifidogenen Peptides aus Milch

Für die Reinigung von bifidogenen Peptiden aus Milch müssen verschiedene HPLC Trennverfahren miteinander kombiniert werden, um eine möglichst reine Darstellung über eine optimale Trennleistung zu erzielen und inaktive, unerwünschte Bestandteile abzutrennen. Die jeweiligen Proben, die nach jedem Trennungsschritt entstehen, müssen in zwei Testsystemen getestet werden, d.h. ein Wachstumstest mit Bifidobakterien in Kombination mit einem Wachstumstest mit E. coli als Target (siehe Beispiele 3 und 4). Notwendig für die Reinigung ist die Kombination von mindestens einem Reverse-Phase Chromatographieschritt (bevorzugt von zwei Reverse-Phase Chromatographischritten) und der Einsatz einer Kationenaustausch-HPLC-Trennung. In den Biotest muss die jeweilige Probe salzarm eingesetzt werden, um ein möglichst optimales Screeningergebnis zu erhalten.

Der erste Trennschritt wurde mit Hilfe einer Parcosil-C18 Säule (1 x 12,5 cm, 100 Å, Biotek, Heidelberg, Deutschland) durchgeführt.
- Puffer A:: 0,1% TFA
- Puffer B:: Acetonitril mit 0,1% TFA
- Gradient:: 0 bis 60% B in 45 Minuten
- Fluss:: 2 ml / min

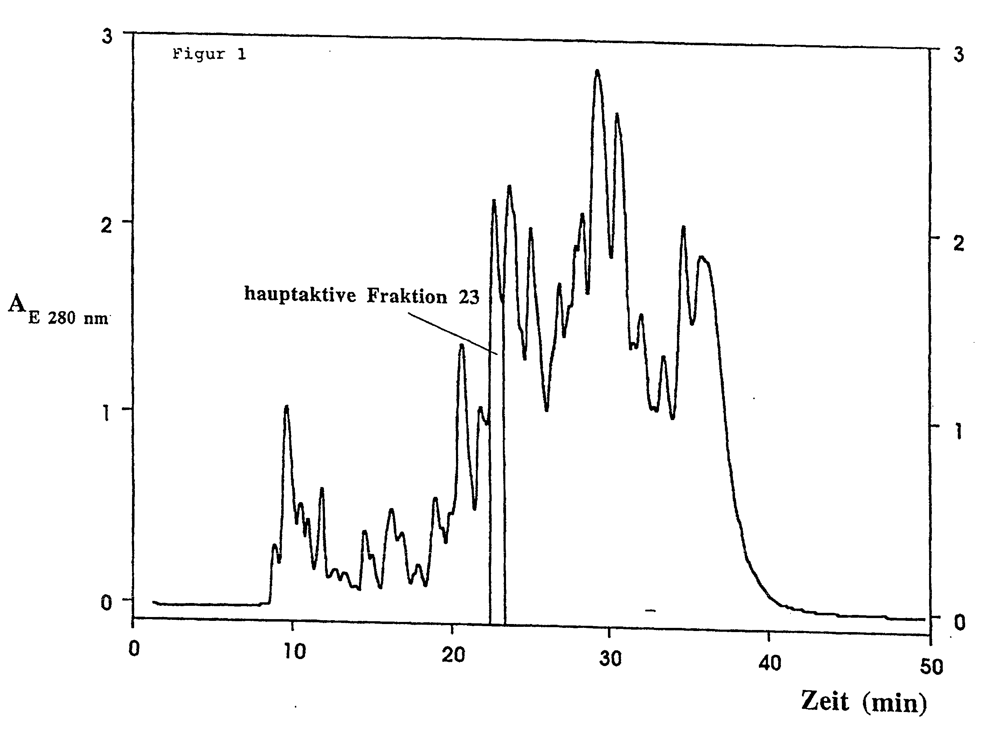

### Detektion 280 nm (siehe Figur 1)

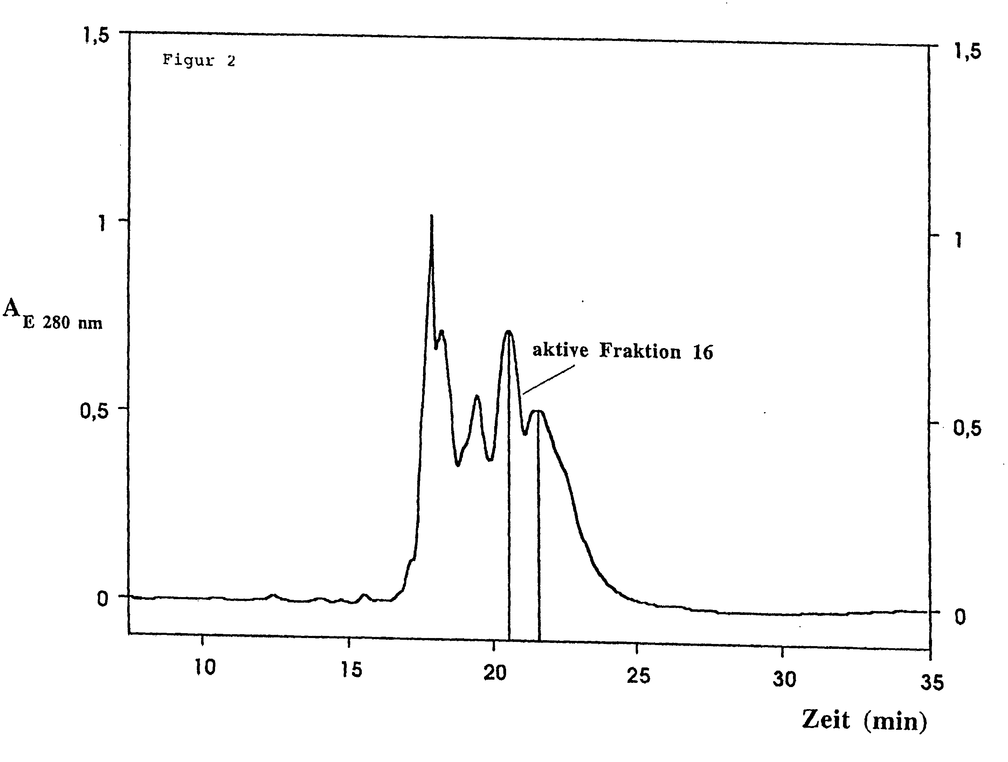

Rechromatografie von Fraktion 23 mit der gleichen Säule und einem flacheren Gradient (siehe Bild):
- Puffer A:: 0,1% TFA
- Puffer B :: Acetonitril mit 0,1% TFA
- Gradient:: 0 bis 20% B in 5 Minuten 20 bis 50% B in 45 Minuten
- Detektion:: 214 nm (siehe Figur 2)

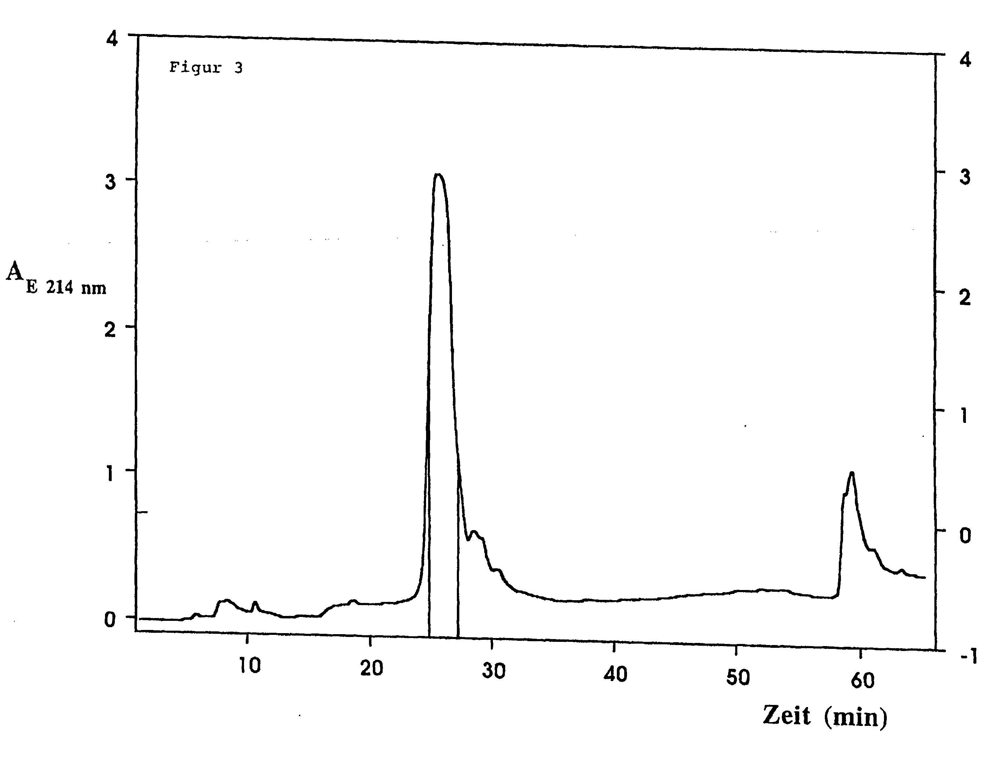

Rechromatografie von Fraktion 16 aus dem vorangegangenem Trennschritt mit der gleichen Säule, aber anderem Elutionsmittel, um die Selektivität bei der Trennung zu verändern.
- Puffer A:: 0,1% TFA
- Puffer B:: 0,1% TFA in Methanol
- Gradient:: 0 bis 40% B in 5 Minuten 40 bis 70% B in 45 Minuten
- Detektion:: 214 nm (siehe Figur 3)

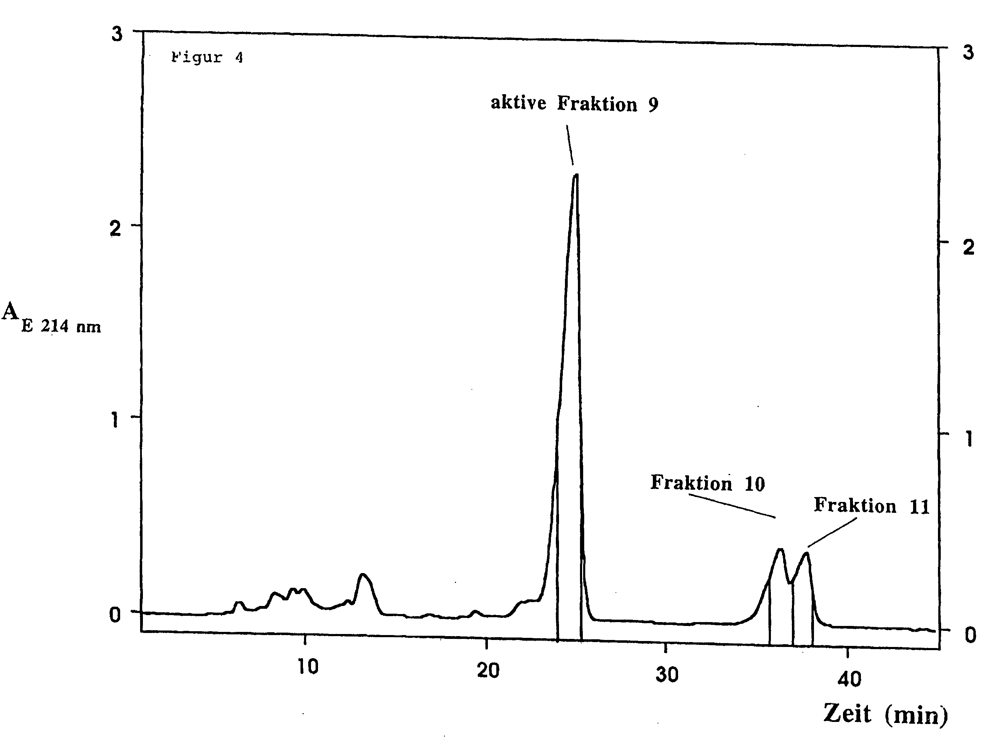

Rechromatografie der aktiven Fraktion 21 mit einer Kationenaustausch HPLC:
- Säule:: Parcosil Pepkat, 4 × 50 mm, 300 A, 5 mm, Biotek, Heidelberg
- Puffer A:: 10 mM Phosphatpuffer pH 4,5
- Puffer B:: Puffer A mit 1 M NaCl
- Fluss:: 0,75 ml/min
- Gradient:: 0 bis 15% B in 5 Minuten 15 bis 50% B in 35 Minuten
- Detektion:: 214 nm (siehe Figur 4)

Die erhaltenen Fraktionen wurden jede einzeln in einem kurzen Reverse Phase HPLC Lauf entsalzt, bevor sie zum Test auf antimikrobielle und bifidogene Aktivität zugeführt wurde.

Durch Massenspektrometrie und Aminosäuresequenzierung wurden die folgenden Peptide identifiziert:
Fraktion 9 enthielt die reine, bifidogene Komponente
(caseinK-63-117)
Fraktion 10 enthält die bifidogene Komponente
(neutrophil-lactoferrin-20-67)
und Fraktion 11 enthält die bifidogene Komponente mit einer Adduktmasse von +16 was darauf hindeutet, dass es sich um ein Oxidationsprodukt handelt (vermutlich ist ein Methionin oxidiert).

Beide Peptide und das Oxidationsprodukt haben eine bifidogene Aktivität.

### Referenzbeispiel 2

### Nachweis der wachstumsregulierenden Aktivität auf E. coli

Fraktionen aus der HPLC wurden mit *E. coli* K12 eingesetzt. Der Test wird in 3 g/l Tryptic Soy broth (Sigma) folgendermaßen durchgeführt:

Für jeden Assay wurden Kulturen von E. coli K12 frisch in Tryptic Soy broth (Firma Sigma, Deisenhofen, Deutschland, Bestellnummer T8907) angeimpft (Difco Manual, 10^{th} Ed., S. 1027). Die Inkubation dieser Bakterien erfolgte immer unter aeroben Bedingungen bei 37°C für 16 Stunden. Zu testende Peptide wurden zu einer Testlösung, bestehend aus 200 ml 3 g/l Tryptic Soy broth, in 96-Loch Zellkulturplatten gegeben und mit einem Inokulum von 20 ml einer verdünnten Bakteriensuspension angeimpft. Die photometrische Absorption des Inokulums betrug 0,05, gemessen bei 500 nm. Das Wachstum der Bakterien unter dem Einfluss der Peptide wurde nach 16 Stunden ebenfalls photometrisch im ELISA-Reader bestimmt und manuell mikroskopisch bestimmt.

### Referenzbeispiel 3

### Nachweis der wachstumsregulierenden Aktivität auf Bifidobacterium bifidum

Für jeden Assay wurden Kulturen von Bifidobacterium bifidum ATCC 29521 frisch in Elliker broth (Difco, Detroit, USA) (Tryptone 20 g, Yeast Extract (Hefeextrakt) 5 g, Gelatin (Gelatine) 2,5 g, Dextrose 5 g, Lactose (Laktose) 5 g, Saccharose 5 g, Sodium Chloride (NaCl) 4 g, Sodium-Acetate (Natriumacetat) 1,5 g, Ascorbic Acid (Ascorbinsäure) 0,5 g) angeimpft. Die Inkubation dieser Bakterien erfolgte immer unter anaeroben Bedingung bei 37°C für 16 bis 18 Stunden. Zu testende Peptide wurden zu einer Testlösung bestehend aus 200 ml 50% Elliker broth in 96-Loch Zellkulturplatten gegeben und mit einem Inokulum von 20 ml einer verdünnten Bakteriensuspension angeimpft. Die photometrische Absorption des Inokulums betrug 0,05 gemessen bei 550 nm. Das Wachstum der Bakterien unter dem Einfluss der Peptide wurde nach 16 Stunden ebenfalls photometrisch im ELISAReader bestimmt und manuell mikroskopisch bestimmt. Als Positivkontrolle diente N-acetylglucosamin. Für diesen Test können nur Bifidus-Kulturen eingesetzt werden, die auf N-Acetylglucosamin reagieren. Nach einigen Passagen verlieren Bifidobakterien diese Eigenschaft, diese können dann für diesen Wachstumstest nicht mehr eingesetzt werden.

## Patentansprüche

1. Peptide ausgewählt aus der Gruppe bestehend aus:
R¹-EVAARARVVW-R²,
R¹-ARRARVVWAAVG-R²,
R¹-ARRARVVWCAVGE-R², 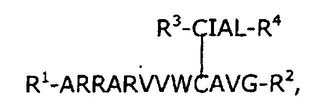
worin
R¹, R³ unabhängig voneinander NH₂, eine Aminosäure oder ein Peptid mit bis 100 Aminosäuren darstellen und
R², R⁴ unabhängig voneinander COOH, CONH₂, eine Aminosäure oder ein Peptid mit bis zu 100 Aminosäuren darstellen
sowie die amidierten, acetylierten, sulfatierten, phosphorylierten, glycosylierten, oxydierten Derivate oder Fragmente mit bifidogenen Eigenschaften und Peptide, die durch Kombination der Peptide, Fragmente oder Derivate mittels chemischer Verknüpfung erhältlich sind.

2. Peptide gemäß Anspruch 1 mit den Seq. ID Nr. 8, 14, 16 und 22.

3. Arzneimittel enthaltend mindestens ein Peptid gemäß Anspruch 1 oder 2.

4. Verwendung von Peptiden gemäß Anspruch 1 oder 2 zur Herstellung eines Arzneimittels zur Behandlung von durch mikrobielle Fehlbesiedlungen bedingten Erkrankungen beispielsweise durch Bakterien, Pilze, Hefen, Protisten, Viren, Mycoplasmen, Filarien, Plasmodien, wie Infektionen, Entzündungen, mikrobiell induzierten Tumoren, mikrobiell bedingten degenerativen Erkrankungen, Durchfallerkrankungen, Koliken, Abweichung der Mund-, Darm- und Vaginalflora, Karies.

5. Verwendung von Nukleinsäuren kodierend für die im Anspruch 1 oder 2 genannten Peptide zur Herstellung eines Arzneimittels zur Behandlung von durch mikrobielle Fehlbesiedlungen bedingten Erkrankungen beispielsweise durch Bakterien, Pilze, Hefen, Protisten, Viren, Mycoplasmen, Filarien, Plasmodien, wie Infektionen, Entzündungen, mikrobiell induzierten Tumoren, mikrobiell bedingten degenerativen Erkrankungen, Durchfallerkrankungen, Koliken, Abweichung der Mund-, Darm- und Vaginalflora, Karies.

## Claims

1. Peptides selected from the group consisting of
R¹-EVAARARVVW-R²,
R¹-ARRARVVWAAVG-R²,
R¹-ARRARVVWCAVGE-R², 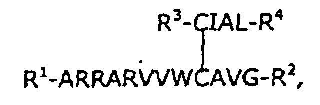
wherein
R¹, R³ independently represent NH₂, an amino acid or a peptide containing up to 100 amino acids; and
R², R⁴ independently represent COOH, CONH₂, an amino acid or a peptide containing up to 100 amino acids;
and the amidated, acetylated, sulfated, phosphorylated, glycosylated, oxidized derivatives or fragments thereof having bifidogenic properties, and peptides obtainable by the combination of the peptides, fragments or derivatives by chemical bonding.

2. The peptides according to claim 1 having the SEQ ID Nos. 8, 14, 16 and 22.

3. A medicament containing at least one peptide according to claim 1 or 2.

4. Use of peptides according to claim 1 or 2 for preparing a medicament for the treatment of diseases caused by misplaced microbial colonizations, for example, by bacteria, fungi, yeasts, protists, viruses, mycoplasmas, filariae, plasmodiums, such as infections, inflammations, microbially induced tumors, microbially caused degenerative diseases, diarrheic diseases, colics, deviations in the oral, intestinal and vaginal floras, caries.

5. Use of nucleic acids coding for the peptides mentioned in claim 1 or 2 for preparing a medicament for the treatment of diseases caused by misplaced microbial colonizations, for example, by bacteria, fungi, yeasts, protists, viruses, mycoplasmas, filariae, plasmodiums, such as infections, inflammations, microbially induced tumors, microbially caused degenerative diseases, diarrheic diseases, colics, deviations in the oral, intestinal and vaginal floras, caries.

## Revendications

1. Peptides sélectionnés parmi le groupe formé par :
R¹-EVAARARVVW-R²,
R¹-ARRARVVWAAVG-R²,
R¹-ARRARVVWCAVGE-R². 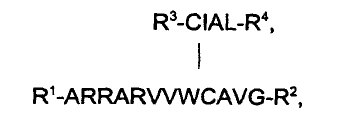
dans lesquels
R¹ et R³ représentent indépendamment l'un de l'autre NH₂, un acide aminé ou un peptide comportant jusqu'à 100 acides aminés et
R² et R⁴ représentent indépendamment l'un de l'autre COOH, CONH₂, un acide aminé ou un peptide comportant jusqu'à 100 acides aminés
ainsi que leurs dérivés amidifiés, acétylés, sulfatés, phosphorylés, glycosylés, oxydés ou des fragments d'entre eux ayant des propriétés bifidogènes et les peptides obtenus par combinaison de peptides, fragments, ou dérivés au moyen d'une liaison chimique.

2. Peptides selon la revendication 1 présentant les séquences de numéros d'identification (SEQ. ID.) N° 8,14, 16 et 22.

3. Médicament contenant au moins un peptide selon la revendication 1 ou 2.

4. Utilisation de peptides selon la revendication 1 ou 2 pour préparer un médicament destiné au traitement des maladies dues à une mauvaise colonisation microbienne, par exemple à cause de bactéries, de champignons, de levures, de protistes, de virus, de mycoplasmes, de filaires, de plasmodia, telles que les infections, les inflammations, les tumeurs induites par voie microbienne, les maladies dégénératives induites par voie microbienne, les diarrhées, les coliques, les aberrations de la flore buccale, intestinale ou vaginale, et les caries.

5. Utilisation d'acides nucléiques codant pour les peptides cités dans la revendication 1 ou 2 pour préparer un médicament destiné au traitement des maladies dues à une mauvaise colonisation microbienne, par exemple à cause de bactéries, de champignons, de levures, de protistes, de virus, de mycoplasmes, de filaires, de plasmodia, telles que les infections, les inflammations, les tumeurs induites par voie microbienne, les maladies dégénératives induites par voie microbienne, les diarrhées, les coliques, les aberrations de la flore buccale, intestinale ou vaginale, et les caries.
